# EUROPEAN PATENT SPECIFICATION

(11) **EP 2 214 488 B1**
(45) Date of publication and mention of the grant of the patent: **21.12.2016**
(21) Application number: 08846840.0
(22) Date of filing: 06.11.2008
(51) Int. Cl.: A61K 31/565, A01N 45/00, A61K 31/568, A61P 25/16

(54) **COMPOSITIONS AND METHODS FOR TREATING PARKINSON'S DISEASE AND RELATED DISORDERS**
ZUSAMMENSETZUNGEN UND VERFAHREN ZUR BEHANDLUNG VON MORBUS PARKINSON UND VERWANDTEN ERKRANKUNGEN
COMPOSITIONS ET PROCÉDÉS POUR TRAITER LA MALADIE DE PARKINSON ET DES TROUBLES ASSOCIÉS

(30) Priority: 06.11.2007 US 2110 P
(43) Date of publication of application: 11.08.2010
(73) Proprietor: Signal Coordinating Therapy, Inc., Pendleton, SC 29670 (US)
(72) Inventor: KLINE, Ellis L., Pendleton, SC 29670 (US)
(74) Representative: Tombling, Adrian George
(86) International application number: PCT/US2008/012510
(87) International publication number: WO 2009/061428

(56) References cited:
- WO-A1-02/069975
- WO-A2-02/087552
- US-A- 4 897 389
- US-A1- 2004 111 127
- US-A1- 2007 198 063
- KLINE E L ET AL: "INHIBITION OF IMIDAZOLE-INDUCED TYROSINASE ACTIVITY BY ESTRADIOL AND ESTRIOL IN CULTURED B16-C3 MELANOMA CELLS", JOURNAL OF CELLULAR PHYSIOLOGY, vol. 134, no. 3, 1988, pages 497-502, ISSN: 0021-9541
- KLINE E L ET AL: "EFFECT OF TESTOSTERONE ON IMIDAZOLE-INDUCED TYROSINASE EXPRESSION IN B16 MELANOMA CELL CULTURE", CANCER RESEARCH, vol. 48, no. 13, 1988, pages 3586-3590, ISSN: 0008-5472
- BOURQUE MÉLANIE ET AL: "Male/Female differences in neuroprotection and neuromodulation of brain dopamine.", FRONTIERS IN ENDOCRINOLOGY 2011, vol. 2, 2011, page 35, ISSN: 1664-2392
- ALYEA REBECCA A ET AL: "Nongenomic mechanisms of physiological estrogen-mediated dopamine efflux.", BMC NEUROSCIENCE 2009, vol. 10, 2009, page 59, ISSN: 1471-2202

## Description

### TECHNICAL FIELD

The present disclosure relates generally to compositions for treating symptoms of Parkinson's disease and/or other movement disorders. More particularly, the present disclosure describes a composition for enhancing or optimizing the effectiveness of neural stimulation in treating the symptoms of movement disorders such as Parkinson's disease.

### BACKGROUND OF THE INVENTION

A wide variety of mental and physical processes are controlled or influenced by neural activity in particular regions of the brain. For example, various physical or cognitive functions are directed or affected by neural activity within the sensory or motor cortices. Across most individuals, particular areas of the brain appear to have distinct functions. In the majority of people, for example, the areas of the occipital lobes relate to vision; the regions of the left interior frontal lobes relate to language; portions of the cerebral cortex appear to be consistently involved with conscious awareness, memory, and intellect; and particular regions of the cerebral cortex as well as the basal ganglia, the thalamus, and the motor cortex cooperatively interact to facilitate motor function control.

Many problems or abnormalities with body functions can be caused by damage, disease, and/or disorders in the brain. For example, Parkinson's disease is related to the degeneration or death of dopamine producing neurons in the substantia nigra region of the basal ganglia in the brain. Dopamine is a neurotransmitter that transmits signals between areas of the brain. As the neurons in the substantia nigra deteriorate, the reduction in dopamine causes abnormal neural activity that results in a chronic, progressive deterioration of motor function control. Conservative estimates indicate that Parkinson's disease may affect more than one million individuals in the United States alone.

Parkinson's disease patients typically exhibit one or more of four primary symptoms. One primary symptom is a tremor in an extremity (e.g., a hand) that occurs while the extremity is at rest. Other primary symptoms include a generalized slowness of movement (bradykinesia); increased muscle rigidity or stiffness (rigidity); and gait or balance problems (postural dysfunction). In addition to or in lieu of these primary symptoms, Parkinson's disease patients may exhibit secondary symptoms including difficulty initiating or resuming movements; loss of fine motor skills; lack of arm swing on the affected side of the body while walking; foot drag on the affected side of the body; decreased facial expression; voice and/or speech changes; cognitive disorders; feelings of depression or anxiety; and/or other symptoms.

Effectively treating Parkinson's disease or other movement disorders related to neurological conditions can be very difficult. Current treatments for Parkinson's disease symptoms include drugs, ablative surgical intervention, and/or neural stimulation. Drug treatments or therapies may involve, for example, the administration of a dopamine precursor that is converted to dopamine within the central nervous system (i.e., Levodopa (L-dopa)). Other types of drug therapies are also available. Unfortunately, drug therapies frequently become less effective or ineffective over time for an undesirably large patient population. A Parkinson's disease patient may require multiple drugs in combination to extend the time period of efficacy of drug therapies. Drug treatments additionally have a significant likelihood of inducing undesirable physical side effects; motor function complications, such as uncontrollable involuntary movements (dyskinesias), are a particularly common side effect. Furthermore, drug treatments may induce undesirable cognitive side effects, such as confusion and/or hallucinations.

Ablative surgical intervention for Parkinson's disease typically involves the destruction of one or more neural structures within the basal ganglia or thalamus that have become overactive because of the lack of dopamine. Unfortunately, such neural structures reside deep within the brain, and hence ablative surgical intervention is a very time consuming and highly invasive procedure. Potential complications associated with the procedure include risk of hemorrhage, stroke, and/or paralysis. Moreover, because Parkinson's disease is a progressive disease, multiple deep brain surgeries may be required as symptoms progressively worsen over time. Although ablative surgical intervention may improve a Parkinson's disease patient's motor function, it is not likely to completely restore normal motor function. Furthermore, since ablative surgical intervention permanently destroys neural tissue, the effects of such intervention cannot be readily adjusted or "fine tuned" over time.

Neural stimulation treatments have shown promising results for reducing some of the symptoms associated with Parkinson's disease. Neural activity is governed by electrical impulses or "action potentials" generated in and propagated by neurons. While in a quiescent state, a neuron is negatively polarized and exhibits a resting membrane potential that is typically between -70 and -60 mV. Through chemical connections known as synapses, any given neuron receives excitatory and inhibitory input signals or stimuli from other neurons. A neuron integrates the excitatory and inhibitory input signals it receives, and generates or fires a series of action potentials in the event that the integration exceeds a threshold potential. A neural firing threshold, for example, may be approximately -55 mV. Action potentials propagate to the neuron's synapses and are then conveyed to other synaptically connected neurons.

Neural activity in the brain can be influenced by neural stimulation, which involves the application of electrical and/or magnetic stimuli to one or more target neural populations within a patient using a waveform generator or other type of device. Various neural functions can thus be promoted or disrupted by applying an electrical current to one or more regions of the brain. As a result, researchers have attempted to treat certain neurological conditions, including Parkinson's disease, using electrical or magnetic stimulation signals to control or affect brain functions.

Deep Brain Stimulation (DBS) is a stimulation therapy that has been used as an alternative to drug treatments and ablative surgical therapies. In DBS, one or more electrodes are surgically implanted into the brain proximate to deep brain or subcortical neural structures. For treating Parkinson's disease or other movement disorders, the electrodes are positioned in or proximate to the ventrointermediate nucleus of the thalamus; basal ganglia structures such as the globus pallidus internalis (GPi); or the Subthalamic Nucleus (STN). The location of the stimulation site for the electrodes depends upon the symptoms that a patient exhibits and the severity of the symptoms.

In a typical DBS system, a pulse generator delivers a continuous or essentially continuous electrical stimulation signal having a pulse repetition frequency of approximately 100 Hz to each of two deep brain electrodes. The electrodes are bilaterally positioned on the left and right sides of the brain relative to particular neural structures such as those indicated above. U.S. Pat. No. 5,883,709 discloses one conventional DBS system for treating movement disorders.

Although DBS therapies may significantly reduce one or more Parkinson's disease symptoms, particularly when combined with drug treatments, they are highly invasive procedures. In general, configuring a DBS system to properly function within a patient requires two time consuming, highly invasive surgical procedures for implanting the DBS electrodes. Each such surgical procedure has essentially the same risks as those described above for ablative surgical intervention. Moreover, DBS may not provide relief from some movement disorders.

Motor Cortex Stimulation (MCS) is another type of brain stimulation treatment that has been proposed for treating movement disorders. MCS involves the application of stimulation signals to the motor cortex of a patient. One MCS system includes a pulse generator connected to a strip electrode that is surgically implanted over a portion of only the motor cortex (precentral gyrus). The use of MCS to treat Parkinson's disease symptoms is described in Canavero, Sergro, Extradural Motor Cortex Stimulation for Advanced Parkinson's Disease: Case Report, Movement Disorders (Vol. 15, No. 1, 2000).

US 4,897,389 discloses the use of estradiol, estrone or estriol for the treatment of Parkinson's disease at a concentration of 10⁻⁹ to 10⁻¹¹ molar.

WO02/069975 teaches the use of estrogen to increase neural stem cells. No suitable concentration is disclosed.

What are needed are compositions and methods that can be safely and easily administered to a Parkinson's disease patient and relieve the symptoms of the disease.

### SUMMARY OF THE INVENTION

The present invention provides compositions for use in the treatment of Parkinson's disease and related movement disorders. The present invention comprises sublingual administration to the patient with Parkinson's disease a composition comprising an active agent at between approximately 10⁻⁹ to approximately 10⁻¹² molar, wherein the active agent comprises:

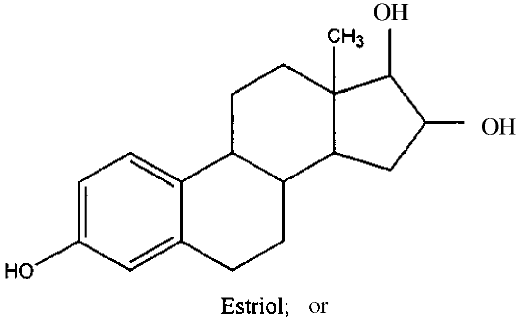

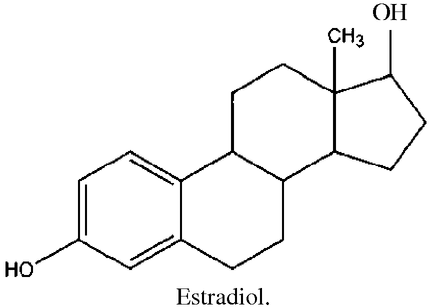

The present invention provides a composition for use in the treatment of Parkinson's disease and related disorders. Typically, a pharmaceutical dosage unit of the present invention for the delivery of the active agent (estradiol or estriol) at a signaling concentration comprises a pharmaceutically acceptable liquid or solid carrier and an effective amount of an effective concentration of the active agent. The aforesaid effective concentration is between approximately 10⁻⁹ to about 10⁻¹² molar, and still more preferably about 3 X 10⁻⁹ to 3 X 10⁻¹⁰ molar active agent in the dosage unit in association with pharmaceutically acceptable excipients. The preferred carrier is sodium chloride (USP) although other excipients well known to those of ordinary skill in the art can be used. A volume of a dose for sublingual administration is between approximately 0.001 ml and 0.5 ml, with the preferred volume being approximately .0025 ml to 0.1 ml, with a most preferred volume being between approximately 0.01 ml to 0.05 ml.

In practice, the present invention comprises the administration of a volume of the signaling compound not to exceed approximately 0.5 ml, although, in certain cases, the total amount of active agent administered in any one day may exceed the preferred limit. The active agent can be administered as a liquid or it can be administered as a solid, wherein the active agent is embedded or admixed in a biodegradable or bioerodible matrix. The matrix can be a time release matrix. These matrices are well known to those of ordinary skill in the art and are not critical to the present invention. The active agent can be administered by injection or by sublingual route. In one embodiment, the vehicle is an aqueous solution that is contained within an inert container. In another variation, the composition is in the form of a suppository. The route of administration is sublingual.

The dosage units can be either liquid or solid. Typically, the dosage unit may be administered up to about 1 to 8 times per day or intermittently depending on the individual case. A typical dosing regimen is to administer the active agent as described herein. In most cases, the symptoms of Parkinson's disease will markedly decrease. When the symptoms reoccur, the active agent is administered again. The compositions may also be formulated to include a pharmaceutically acceptable excipient, diluent, or carrier.

The active agents may be administered individually or may be administered in any combination thereof. When administered in combination, the active agents may be administered simultaneously or sequentially. For example, estradiol and estriol may be administered simultaneously as a single composition, or administered sequentially as two separate compositions.

In one embodiment the compositions of the present invention are administered as a signaling solution in conjunction with standard neural stimulation therapies. Examples of neural stimulation therapies include, but are not limited to, Deep Brain Stimulation (DBS) and Motor Cortex Stimulation (MCS). The signaling solution may be administered prior to, concurrently with, or after neural stimulation therapy. The signaling solution of the present invention may enhance or optimize the effectiveness of neural stimulation by improving the response to neural stimulation therapy as measured by a further reduction of symptoms of Parkinson's disease and related movement disorders, or an increase in the symptom free period (off time) between neural stimulation therapy treatments. The signaling solution of the present invention may also decrease the intensity and length of the neural stimulation treatment.

These and other objects, features, and advantages will become apparent after a review of the following detailed description of the disclosed embodiments and the appended claims.

### DESCRIPTION OF THE INVENTION

The present invention may be understood more readily by reference to the following detailed description of specific embodiments included herein.

The present invention is directed to compositions for use in treating Parkinson's disease. The compositions of the present invention comprise an active agent in an amount effective to treat one or more symptoms of Parkinson's disease. The active agent is selected from one or more of estradiol or estriol. Methods for assessing a related steroid compound's ability to treat one or more symptoms of movement disorder, such as Parkinson's disease, are described below.

The active agents may be administered individually or may be administered in any combination thereof. In addition, the active agents may be administered in combination with or without neural stimulation therapy. The use of the term "in combination" does not restrict the order in which the active agents are administered to a subject with a disease or disorder. A first active agent can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second active agent or application of neural stimulation therapy to a subject with a disease or disorder.

Examples of estradiol related compounds that may be used in the present invention include estrogenic compounds such as naturally occurring 17 beta-estradiol or esters of 17 beta-estradiol. Examples of estradiol esters include, but are not limited to, estradiol-3,17- diacetate; estradiol-3 -acetate; estradiol- 17-acetate, estradiol-3,17-divalerate; estradiol-3- valerate; estradiol- 17-valerate; 3-mono, 17-mono and 3,17-dipropionate esters, and the corresponding cypionate, heptanoate, benzoate esters. Other estrogen-related compounds that may be used in the present invention include conjugated estrogens (including estrone sulfate, equilin, and 17-α-dihydroequiline), estradiol valerate, estrone, estrone sulfate, estropipate, ethinyl estradiol, mestranol, and all salts, esters, amides and enantiomers thereof. In addition, endogenous metabolites of estrogen such as 2-methoxyestradiol and 4- methoxyestradiol and derivatives, thereof may be used in the compositions of the present invention. A number of 2-methoxyestradiol derivatives that may be useful in the present invention are described in U.S. Patent Application No. 12/262,318.

As used herein, the terms "treat", "treatment" and "treating" refer to the amelioration of one or more of the primary or secondary symptoms of Parkinson's disease and related movement disorders. Primary symptoms of Parkinson's disease include, but are not limited to tremor in an extremity while the extremity is at rest, generalized slowness of movement (bradykinesia), increased muscle rigidity or stiffness, gait or balance problems (postural dysfunction). Secondary symptoms of Parkinson's include, but are not limited to, difficulty initiating or resuming movements, loss of fine motor skills, lack of arm swing on the affected side of the body while walking, foot drag on the affected side of the body, decreased facial expression, voice and/or speech changes, cognitive disorders, and feelings of depression or anxiety.

Related movement disorder that may also be treated by the methods and compositions of the present invention include, but are not limited to, akathisia, akinesia (lack of movement), athetosis (contorted torsion or twisting), ataxia, ballismus (violent involuntary rapid and irregular movements), Cerebral palsy, choreas (e.g. Syndenham's chorea, rheumatic chorea, Huntington's disease), dystonias (e.g. dystonia musularum, belpharospasm, Writer's cramp, spasmodic torticollis), geniospasm (episodic involuntary up and down movements of the chin and lower lip) myoclonus, Restless Legs Syndrome (RLS), spasms, stereotypic movement disorder, stereotypy, Tardive dyskinesia, tic disorders (Tourette's syndrome, postural tremor, kinetic tremor, essential tremor, cerebellar tremor, physiological tremore), and Wilson's disease.

### Pharmaceutical Compositions

The active agents of the present invention may be combined with a pharmaceutically acceptable carrier, excipients, or stabilizer. Examples of pharmaceutically acceptable carriers, excipients and stablizers include, but are not limited to, buffers such as phosphate, citrate, and other organic acids; antioxidants including ascorbic acid; low molecular weight polypeptides; proteins, such as serum albumin and gelatin; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt- forming counterions such as sodium; and/or nonionic surfactants such as TWEEN™, polyethylene glycol (PEG), and PLURONICS™. The pharmaceutical composition of the present invention can also include a lubricant, a wetting agent, a sweetener, a flavoring agent, an emulsifier, a suspending agent, and a preservative, in addition to the above ingredients.

The present invention also relates to conjugated prodrugs and uses thereof. More particularly, the invention relates to conjugates of the active agents, such as estradiol or estriol and the use of such conjugates in the prophylaxis or treatment of conditions associated with movement disorders, such as Parkinson's disease. The invention also relates to compositions including the prodrugs of the present invention. In one aspect, the present invention provides a conjugate of the active agent to a biological activity modifying agent. In another aspect, the present invention provides a conjugate of the active agent to a peptide moiety.

Pharmaceutically acceptable salts of the active agents or the prodrugs thereof, can be prepared in any conventional manner, for example from the free base and acid. In vivo hydrolysable esters, amides and carbamates and other acceptable prodrugs of the active agent can be prepared in any conventional manner.

100% pure isomers are contemplated by this invention; however a stereochemical isomer (labeled as α or β, or as R or S) may be a mixture of both in any ratio, where it is chemically possible by one skilled in the art. Also contemplated by this invention are both classical and non-classical bioisosteric atom and substituent replacements, such as are described by Patani and Lavoie ("Bio-isosterism: a rational approach in drug design" Chem. Rev. (1996) pp. 3147-3176) and are well known to one skilled in the art. Such bioisosteric replacements include, for example, but are not limited to, substitution of =S or =NH for =0.

### Administration

The compositions described herein can be provided as physiologically acceptable formulations using known techniques, and the formulations can be administered by standard routes. In general, the active agents can be administered by topical, oral, rectal, nasal, inhalation or parenteral (e.g., intravenous, subcutaneous, intramuscular, intradermal, intraocular, intratracheal or epidural) routes. In addition, the compositions can be incorporated into polymers allowing for sustained release, the polymers being implanted in the vicinity of where delivery is desired, for example, proximate to the ventrointermediate nucleus of the thalamus, basal ganglia structures such as the globus pallidus internalis (GPi), or the subthalamic nucleus (STN), or the polymers can be implanted, for example, subcutaneously or intramuscularly or delivered intravenously or intraperitoneally to result in systemic delivery of the active agent. Other formulations for controlled, prolonged release of therapeutic agents useful in the present invention are disclosed in U.S. Patent No. 6,706,289.

Formulations contemplated as part of the present invention include nanoparticle formulations made by methods disclosed in U.S. Patent Application No. 10/392,403 (Publication No. 2004/0033267). By forming nanoparticles, the compositions disclosed herein are shown to have increased bioavailability. Preferably, the particles of the compounds of the present invention have an effective average particle size of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy.

The described formulations can be administered in the form of a tablet, a capsule, a lozenge, a cachet, a solution, a suspension, an emulsion, a powder, an aerosol, a suppository, a spray, a pastille, an ointment, a cream, a paste, a foam, a gel, a tampon, a pessary, a granule, a bolus, a mouthwash, or a transdermal patch.

The formulations include those suitable for oral, rectal, nasal, inhalation, topical (including dermal, transdermal, buccal and sublingual), vaginal, parenteral (including subcutaneous, intramuscular, intravenous, intradermal, intraocular, intratracheal, and epidural) or inhalation administration. The formulations can conveniently be presented in unit dosage form and can be prepared by conventional pharmaceutical techniques. Such techniques include the step of bringing into association the active ingredient(s) and a pharmaceutical carrier(s) or excipient(s). In general, the formulations are prepared by uniformly and intimately bringing into association the active ingredient(s) with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient or ingredients; as a powder or granules; as a solution or a suspension in an aqueous liquid or a non-aqueous liquid; or as an oil-in-water liquid emulsion or a water-in-oil emulsion, etc.

A tablet may be made by compression or molding, optionally with one or more accessory ingredients. Compressed tablets may be prepared by compressing, in a suitable machine, the active ingredient in a free-flowing form such as a powder or granules, optionally mixed with a binder, lubricant, inert diluent, preservative, surface-active or dispersing agent. Molded tablets may be made by molding, in a suitable machine, a mixture of the powdered compound or compounds moistened with an inert liquid diluent. The tablets may optionally be coated or scored and may be formulated so as to provide a slow or controlled release of the active ingredient therein.

Formulations suitable for topical administration in the mouth include lozenges comprising the ingredients in a flavored base, usually sucrose and acacia or tragacanth; pastilles comprising the active ingredients in an inert base such as gelatin and glycerin, or sucrose and acacia; and mouthwashes comprising the ingredient to be administered in a suitable liquid carrier.

Formulations suitable for topical administration to the skin may be presented as ointments, creams, gels or pastes comprising the ingredient to be administered in a pharmaceutical acceptable carrier. In one embodiment the topical delivery system is a transdermal patch containing the ingredient to be administered. Formulations for rectal administration may be presented as a suppository with a suitable base comprising, for example, cocoa butter or a salicylate.

Formulations suitable for nasal administration, wherein the carrier is a solid, include a coarse powder having a particle size, for example, in the range of 20 to 500 microns which is administered in the manner in which snuff is taken; i.e., by rapid inhalation through the nasal passage from a container of the powder held close up to the nose. Suitable formulations, wherein the carrier is a liquid, for administration, as for example, a nasal spray or as nasal drops, include aqueous or oily solutions of the active ingredient.

Formulations suitable for vaginal administration may be presented as pessaries, tampons, creams, gels, pastes, foams or spray formulations containing, in addition to the active ingredient, ingredients such as carriers that are known in the art to be appropriate.

Formulations suitable for inhalation may be presented as mists, dusts, powders or spray formulations containing, in addition to the active ingredient, ingredients such as carriers that are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

Formulations suitable for parenteral administration include particulate preparations of the anti-angiogenic agents, including, but not limited to, low-micron, or nanometer {e.g., less than 2000 nanometers, preferably less than 1000 nanometers, most preferably less than 500 nanometers, especially less than 75 nanometers in average cross section) sized particles, which particles are comprised of 2-methoxyestradiol analogs and/or one or more anti-cancer agents alone or in combination with accessory ingredients or in a polymer for sustained release. The formulations may be presented in unit-dose or multi-dose containers, for example, sealed ampules and vials, and may be stored in freeze-dried (lyophilized) conditions requiring only the addition of a sterile liquid carrier, for example, water for injections, immediately prior to use. Extemporaneous injection solutions and suspensions may be prepared from sterile powders, granules and tablets of the kinds previously described.

### Methods for Assessing Activity of Active Agents

There are a number of in vitro and in vivo models which may be used by one of ordinary skill in the art to determine the activity, the optimal dosage and the optimal administration regime, both with or without neural stimulation therapy, of the active agents of the present invention. A suitable animal model for translational research of Parkinson's disease and related movement disorders should induce a replicable nigral lesion and show stable dopaminergic cell-loss over time without spontaneous recovery (Emborg, Journal of Neuroscience Methods. 2004, 139:121-143). Emborg provides an excellent overview of the various models that may be used, a summary of the primary models and methodology is provided below.

The neurotoxin 6-hydroxydopamine (6-OHDA) was the first compound discovered to induce selective catecholaminergic cell death (Jonsson and Sachs, J. Nerurochem. 1975, 25:509-16, Thoenen and Tranzer, Nauyn Schmeidebergs Arch Exp Pathol Pharkaol. 1968, 261:271-88 and Ungerstedt Eur. J. Pharmacol. 1968, 5:107-10). 6-OHDA induces specific damage via oxidative stress, by using the catecholamine transport system. 6-OHDA must be administered intracerebrally to exert its toxic effects as it is unable to cross the blood brain barrier. This experimental parkinsonian model has been produced in many species, including mice, rats, cats, dogs and non-human primates (Eslamboli et al. Exp Neurol 2003, 183:418- 29) and Zigmond and Strieker, Brain Research. 1989, 31:1-79). The rat is the most commonly used species due to the replicability and stability of the induced model. Specific target areas can be selected using a stereotaxic atlas (Paxinos and Watson. The rat brain in stereotaxic coordinates. 2<nd> Ed. San Diego Academic Press, 1986). 6-OHDA must be freshly prepared prior to administration. This is typically done by dissolving the powder in saline and ascorbic acid (0.2 mg/ml in 0.9% saline) usually at a concentration of 5 µg/ml. The injections are typically made in the medial forebrain bundle, the substantia nigra or into the striatum. (Ungerstedt, Ungerstedt and Arbuthnott, Brain Research. 1970, 24:485-93 and Kirik et al. Exp Neurol. 1998, 152:259-77) The time course of progression and severity of the lesions can be controlled by location site, number of injection sites and amount of neurotoxin. Middle forebrain bundle, nigral or four instrastriatal injections induce lesions associated with behavioral impairments. Middle forebrain bundle or nigral targets induce and almost complete nigal lesion representing end stage Parkinson's disease. The four site intrastriatal model induces severe lesion (>70% nigral cell loss) analogous to a symptomatic stage of Parkinson's disease.

MPTP is a mitochondrial complex I inhibitor that was discovered in humans who where accidentally exposed to the compound (Davis et al. Psychiatry Res. 1979, 1:249-54, Langston et al. Science. 1983, 219:979-80). Unlike 6-OHDA, MPTP is highly lipophilic and readily crosses the blood brain barrier. MPTP is transformed into its toxic MPP+ by the enzyme MAO-B. MPP+ is then transported into dopaminergic neurons by the DA transporter system. MPTP can be administered orally and by intracerebral stereotaxic injections with the most reliable and replicable lesions induced by the systemic (intravenous, subcutaneous, etc.) or intracarotid artery injection of freshly prepared MPTP solution. The most frequently used animals are mice and monkeys. Rats are relatively insensitive to MPTP (Giovanni et al. J Pharmacol Exp Ther. 1994, 270:1008-14). MPTP is usually administered to mice in two schemes: "acute" (20mg/kg i.p., four injections at 2 hr intervals in 1 day) or "chronic" (30 mg/kg i.p. once a day for 5 days). The acute regime results in nigral cell death mainly by necrotic mechanisms and has higher mortality than the chronic treatment but does allow behavioral deficients to be detected by specific tests (Jackson-Lewis et al. Neurodegeneration. 1995, 4:257-69, Przedborksi and Vila, Ann NY Acad Sci. 2003, 991:189-98, and Vila et al. J Neurochem. 2000, 74:721-9). The chronic administration induces cell death primarily through apoptotic mechanisms but almost undetectable behaviorial deficits (Tatton and Kish, Neuroscience. 1997, 77:1037-48). For monkeys, multiple systemic injections (Burns et al. Proc Natl Acad Sci USA. 1983, 80:4546-50 and Langston et al. Brain Research. 1984, 292:390-4) or intracarotid infusion (Bankiewicz et al. Life Sci. 1986, 39:7-16) are primarily used. Combinations of systemic and intracarotid (Eberling et al. Brain Research. 1998, 805:259-62, and Oiwa et al. Front Biosci. 2003, 8:a155-66) or bilateral intracarotid administration (Smith et al. Neuroscience. 1993, 52:7-16) have been attempted to affect the characteristics and speed of onset. Monkeys in general are more sensitive than rodents to MPTP and their dosing has to be adjusted accordingly. Administration of 0.3-0.4 mg/kg i.m. of MPTP for five consecutive days induces an acute and severe dopaminergic nigral cell loss (Taylor et al. Brain. 1990, 113(Part3):617-37 and Taylor et al. Behav Neurosci. 1990, 104:564-76). An alternative dosing is the administration of MPTP one to two times per week, for many weeks or months, as needed to develop the desired level of disability (Hantraye et al. Neuroscience. 1993, 53:169-78 and Langston et al. Ann Neurol. 2000, 47:S70-89, and Perez-Otano et al. Neurosci Lett. 1994, 175:121-5). This more time consuming regimen induces a slower protracted generation, with less mortality and individual variability.

For purposes of evaluating the ability of the active agents of the present invention to enhance or optimize the overall effectiveness of neural stimulation therapy, a number of rodent models of deep brain stimulation may be used. It has been demonstrated in rodent models that implanting microelectrodes in the STN and applying stimulation parameters similar to those used in humans can improve motor activity (Chang et al. Brain Research. 2003, 983:174-184, Darbaky et al. Eur J of Neurosci. 2003, 25:7687-96, Shi et al. Brain Research. 2004 1013:98-106, and Temel et al. Exp Neurol. 2005, 193:43-52). Chang et al. (Neuroscience and Biobehavioral Reviews. 2008, 32:352-366) provide an overview of the current methodologies used in deep brain stimulation rodent models of Parkinson's disease. These models can be used to assess the ability of the active agents of the present invention to enhance or optimize the overall effectiveness of neural stimulation therapy by administering the active agents in combination with deep brain stimulation.

### Neural Stimulation Therapy

There are a number of neural stimulation therapy techniques that have been shown to be useful in treating movement disorders, such as Parkinson's disease. The following are exemplary embodiments of neural stimulation therapies with which the compositions of the present invention may used to optimize or enhance the overall effectiveness of the treatment. One of ordinary skill in the art would recognize that other methods that rely on the general principle of neural stimulation in order to counter the effects of dopaminergeric cell loss in movement disorders may also be used with the compositions of the present invention.

Deep brain stimulation is an invasive surgical technique involving the implantation of a medical device referred to as a brain pacemaker. The deep brain stimulation system consists of three components; the implanted generator, the lead, and the extension. The implanted generator is a battery-powered neurostimulator encased in titanium housing, which sends electrical signals to the brain to interfere with neural activity. The lead is a coiled wire containing multiple electrodes and is connected to the implanted generator by the extension. The placement of the lead is dependent on the symptoms to be addressed. For non- Parkinsonian essential tremor the lead is placed in the ventrointermedial nucleus (VIM) of the thalamus. For dystonia and symptoms associated with Parkinson's disease, the lead may be placed in either the globus pallidus or subthalamic nucleus. The direct effects of DBS on the physiology of brain cells and neurotransmitters is currently debated, but by sending high frequency electrical impulses into specific areas of the brain it can mitigate symptoms of Parkinson's disease and other movement disorders (Moro et al. Expert Review of Neurotherapeutics. 2006, 6(11): 1695-1705). Because there are multiple sites within the brain that the electrodes may be placed, each with differing results, each patient must be assessed individually to determine the optimal placement of the leads. The goal of DBS treatment is to provide stimulating impulses only when they are needed to prevent tremors and other symptoms before they start.

Motor cortex stimulation (MCS) is a well known treatment option for neuropathic drug-resistant pain and possibly associated movement disorders. Preliminary studies suggest the ability to treat symptoms of Parkinson's disease (Cioni et al. Clinical Neurophysiology. 2007, 37:441-447). One or more electrodes are placed extradurally over the motor cortex through a burr hole or a small craniotomy, and then connected to an implantable neurostimulator. The use of MCS in treating Parkinson's is still in the preliminary stages and potential side effects or adverse heath effects are not yet characterized. However, like DBS motor cortex stimulation requires an invasive surgical procedure and requires further post- operation optimization.

There are also a number of noninvasive neurostimulatory methods under development for treatment of movement disorders such as Parkinson's. Repetitive transcranial magnetic stimulation (rTMS) is the application of trains of repeated magnetic pulses delivered to the scalp. Passing a brief time-varying current through an insulated coil held parallel to the scalp surface generates a magnetic field perpendicular to the coil. This magnetic field, in turn, induces a weak eddy current within the underlying cerebral cortex. Whereas circular TMS coils induce a relatively nonfocal circular band of stimulation in the brain, focal figure-8 coils can target stimulation with the functional spatial resolution of 0.5 cm to 1 cm. Transcranial direct current stimulation (tDCS) refers to application of a constant direct current through a pair of surface electrodes affixed to the scalp. A low-intensity current (1-2 mA) flows from cathode to anode. The scalp possesses high impedance, but sufficient intracranial current flows to produce changes to membrane resting thresholds within the cortex beneath each electrode. The result is an increase in activity under the anode and a decrease in activity under the cathode (Wu et al. Neurotherapeutics, 2008, 5(2): 345-361).

Having described the invention with reference to particular compositions, method for detection, and source of activity, and proposals of effectiveness, and the like, it will be apparent to those of skill in the art that it is not intended that the invention be limited by such illustrative embodiments or mechanisms. It should be understood that any of the above described one or more elements from any embodiment can be combined with any one or more element in any other embodiment. Moreover, when a range is mentioned, it should be understood that it is contemplated that any real number that falls within the range is a contemplated end point. For example, if a range of 0.9 and 1.1 g/kg is given, it is contemplated that any real number value that falls within that range (for example, 0.954 to 1.052 g/kg) is contemplated as a subgenus range of the invention, even if those values are not explicitly mentioned. Finally, the above description is not to be construed to limit the invention but the invention should rather be defined by the below claims.

## Claims

1. A composition comprising an active agent, wherein the active agent is estradiol or estriol, for use in treating a patient with Parkinson's disease by sublingual administration to reduce symptoms of Parkinson's Disease, wherein the active agent is present in a pharmaceutically acceptable excipient at a concentration of between approximately 10⁻⁹ to approximately 10⁻¹² molar.

2. The composition for use according to Claim 1, wherein the active agent is at a concentration between 3 X 10⁻⁹ to 3 X 10⁻¹⁰ molar.

## Patentansprüche

1. Zusammensetzung, die einen Wirkstoff umfasst, wobei der Wirkstoff Estradiol oder Estriol ist, zur Verwendung bei der Behandlung eines Patienten mit Parkinson-Erkrankung durch sublinguale Verabreichung, um die Symptome der Parkinson-Erkrankung zu vermindern, wobei der Wirkstoff in einem pharmazeutisch akzeptablen Hilfsstoff in einer Konzentration von ungefähr 10⁻⁹ bis ungefähr 10⁻¹² molar vorliegt.

2. Zusammensetzung zur Verwendung gemäß Anspruch 1, wobei der Wirkstoff in einer Konzentration von 3 x 10⁻⁹ bis 3 x 10⁻¹⁰ molar vorliegt.

## Revendications

1. Composition comprenant, en tant que principe actif, l'oestradiol ou l'oestriol, pour son utilisation dans le traitement d'un patient atteint de la maladie de Parkinson en vue d'en réduire les symptômes, l'utilisation comprenant l'administration sublinguale de ladite composition, le principe actif étant présent dans un excipient pharmaceutiquement acceptable à une concentration comprise entre environ 10⁻⁹ et environ 10¹² Molaire.

2. Composition selon la revendication 1, **caractérisée en ce que** la concentration dudit principe actif est comprise entre 3 X 10⁻⁹ et 3 X 10⁻¹⁰ Molaire.
